# FASCICULE DE BREVET EUROPEEN

(11) **EP 2 846 870 B1**
(45) Date de publication et mention de la délivrance du brevet: **13.01.2021**
(21) Numéro de dépôt: 13730341.8
(22) Date de dépôt: 08.05.2013
(51) Int. Cl.: A61M 37/00, A61M 5/20, A61M 5/32, A61M 5/158, A61M 5/46

(54) **DISPOSITIF POUR L'INSERTION D'AIGUILLES**
VORRICHTUNG FÜR DAS EINSETZEN VON NADELN
DEVICE FOR INSERTING NEEDLES

(30) Priorité: 10.05.2012 EP 12167545
(43) Date de publication de la demande: 18.03.2015
(73) Titulaire: Debiotech S.A., 1004 Lausanne (CH)
(72) Inventeur: DA ROS, Jérôme, F-74200 Thonon les Bains (FR); NEFTEL, Frédéric, CH-1005 Lausanne (CH)
(74) Mandataire: Weihs, Bruno Konrad
(86) Numéro de dépôt international: PCT/IB2013/053708
(87) Numéro de publication internationale: WO 2013/168107

(56) Documents cités:
- WO-A1-2008/020780
- WO-A1-2012/046816
- WO-A2-2004/019777
- WO-A2-2005/049107
- US-A1- 2010 121 307
- US-A1- 2011 276 027

## Description

### Domaine de l'invention

La présente invention concerne l'insertion d'aiguilles, en particulier de micro-aiguilles.

Celle-ci peut être utilisée pour l'injection intradermique ou sous-cutanée de solutions.

### Etat de la technique

Des dispositifs pour l'insertion de micro-aiguilles sont divulgués dans les documents suivants : US 6 743 211, US 4 886 499, US 7 083 592 et US 20100030148.

US 2011/276027 A1 décrit un applicateur pour une microprojection qui comprend dans un mode de réalisation un élément de stockage de l'énergie. Une application de force case l'élément de stockage de l'énergie comprimé à s'étendre ou à changer d'état entre une première et une deuxième configuration, relachant ainsi l'énergie stockée pour déployer un moyen de retenue dans l'application, qui est configuré pour retenir une séquence de microprojections.

WO 2008/020780 A1 est relative à un seringue jetable qui comprend un corps pourvu d'un piston ayant une cavité, une unité de vérouillage et un arrêt arrangé dedans. Un moyen de ressort est situé entre le corps et le piston.

WO 2004/019777 A1 est relative à un dispositif pour dispenser une substance dans la peau au moyen d'une mouvement de rotation d'un dispositif micro-abrasif, qui permet de réduire les effets variable d'un opérateur. Il est prévu d'exploiter le mouvement de rotation de micro-protubérances.

### Description générale de l'invention

La présente invention est décrite et caractérisée par la revendication indépendante tandis que les revendications dépendantes décrivent d'autres caractéristiques de l'invention.

Dans le cadre de la présente invention, l'expression «extrémité distale» désigne l'extrémité qui est la plus éloignée de la main de l'opérateur et l'expression «extrémité proximale» désigne l'extrémité qui est la plus rapprochée de la main de l'opérateur.

Dans le cadre de la présente invention, l'expression « moyens de pression » désigne au moins un élément ou une combinaison d'éléments ayant la faculté d'exercer une force ou d'appliquer une pression sur lui-même (ou eux-mêmes) et/ou sur un autre élément (ou une autre combinaison d'éléments). Il peut s'agir, de manière non limitative, de l'attraction ou la répulsion entre deux ou plusieurs éléments (par exemple des éléments magnétiques, l'effet de l'attraction terrestre sur un élément du dispositif) et/ou une réaction mécanique (ressort, lame élastique, forme de l'élément, matériaux à mémoire de forme) et/ou autre réaction (réaction chimique, libération d'un gaz comprimé).

Dans le cadre de la présente invention, le terme « tissu » désigne un ensemble de cellules qui remplissent en commun un certain nombre de fonction.

Dans un mode de réalisation possible, le dispositif comprend un boitier, défini par une extrémité distale destinée à venir en contact avec un tissu et une extrémité proximale opposée, dans lequel un piston est monté coulissant et pouvant être restreint à un mouvement dans un axe formant l'orientation principale. Au moins une aiguille creuse fait saillie sur ledit piston afin de pénétrer ledit tissu lorsque ledit piston est à proximité du ou en contact avec le tissu. Ledit piston est mis en mouvement par des moyens de propulsion exerçant une force F1(t) contre ledit piston.

L'invention permet notamment de percuter un tissu par une ou plusieurs micro-aiguilles à des vitesses relativement élevées, typiquement de l'ordre de 3 à 15 m/s (typiquement 7m/s), tout en laissant le tissu amortir le choc sur une certaine distance, ce qui a pour effet d'améliorer la perforation du tissu et de ramener cette dernière à un état stable d'équilibre, minimisant de la sorte les contraintes exercées sur le tissu du patient. Les tissus peuvent avoir des caractéristiques différentes en fonction de leur localisation, âge, type de tissu, genre et/ou espèce (animaux, végétaux, humain). Ainsi, ladite distance parcourue pendant l'amortissement peut être variable voir nulle.

La présente invention permet en particulier de réduire la pression exercée sur la ou les aiguilles (ou de façon plus générale, sur le piston; ce dernier ayant pour fonction de supporter les aiguilles) et par conséquent sur le tissu pendant l'injection de la solution.

Dans un mode de réalisation, une fois insérées, les micro-aiguilles sont maintenues contre le tissu grâce à une légère pression pouvant être différent des moyens de propulsion. Cette légère pression peut éviter le retrait des micro-aiguilles lors de l'insertion et/ou après l'insertion et/ou durant l'injection du fluide. En effet, suite à l'impact des micro aiguilles contre le tissu, le tissu peut exercer une contre force en réaction dudit impact ; cette contre force peut projeter le piston dans le sens opposé de l'insertion et ainsi occasionner un retrait desdites micro-aiguilles du tissu. Ainsi, cette légère pression peut avoir pour objectif de maintenir les microaiguilles au moins partiellement inséré dans le tissu. Cette légère pression peut également permettre de maintenir les micro-aiguilles durant l'injection afin d'éviter toute fuite et/ou tout retrait des micro-aiguilles durant l'injection. Toutefois, cette légère pression ne doit pouvoir s'opposer à la formation de papules induites par l'injection de la solution, c'est-à-dire que la légère pression exercée doit permettre un léger retrait ou déplacement des aiguilles avec le tissu au moment de la formation de cette papule. En particulier cette pression ne doit pas comprimer le tissu de manière trop prononcée, empêchant ainsi (ou limitant trop fortement) le fluide d'y circuler. Dans un mode de réalisation, cette légère pression pourrait permettre au dispositif d'appliquer une force supplémentaire afin de faciliter l'insertion des aiguilles.

Dans un mode de réalisation, cette pression peut être rendue possible grâce à des moyens de pression maintenant les aiguilles sur le tissu une fois celles-ci pénétrés. Ces moyens de pression ne peuvent toutefois être trop importants afin d'autoriser la création de papule durant l'injection.

Dans le cadre de cette invention et dans un exemple ne formant par partie de l'invention d'un mode de réalisation et d'utilisation, l'injection intradermique peut induire la formation d'une papule qui correspond à la déformation du tissu suite au stockage de la solution injectée dans le tissu, notamment lors d'une injection type bolus. A relever que la création et le maintien d'une papule sont souhaitables pour assurer une injection optimale dans le tissu ciblé. C'est pourquoi la présente invention permet à une telle papule de se former et de rester en place le temps nécessaire à la bonne diffusion de la substance injectée.

Décrit mais ne faisant pas part de l'invention est également une méthode d'insertion et d'injection ou de prélèvement grâce à une ou des aiguilles, notamment de micro-aiguilles et notamment dans le derme, où l'aiguille - dotée d'un mouvement de translation selon une direction correspondant à l'orientation principale - peut décélérer soudainement ou progressivement dans le tissu du fait de l'élasticité de celui-ci sur une certaine longueur tout en assurant in fine un maintien de l'aiguille après l'insertion et durant l'injection et/ou durant le prélèvement grâce à une très légère pression de l'aguille contre le tissu. L'ensemble aiguille-piston peut exercer une pression contre le tissu durant l'insertion de telle sorte que l'ensemble aiguille-piston peut s'enfoncer dans le tissu où l'aiguille pénètre le tissu et la face distale du piston pousse le tissu. La profondeur de l'insertion de l'aguille est assurée par ladite face distale du piston limitant la profondeur d'insertion lorsque le piston est en contact avec le tissu. Dans mode de réalisation, le boitier comprend une butée limitant la profondeur d'insertion lorsque le piston est en contact avec ladite butée.

Dans un mode de réalisation, le dispositif selon l'invention offre aussi l'avantage de pouvoir être utilisé dans n'importe quelle orientation par rapport au tissu.

Dans un mode réalisation préféré, le piston et les moyens de propulsion sont maintenu temporairement solidaire grâce des moyens de retenue. Dans un mode de réalisation, les moyens de retenue permettent de rendre solidaire ledit piston aux moyens de propulsion au moins pendant une fraction de temps durant le mis en mouvement des moyens de propulsion. Par exemple mais sans s'y limiter, le propulseur dispose de clips adaptés pour retenir le piston. Lesdits clips sont configurés pour entraîner le piston en direction du tissu grâce au propulseur lors du déclenchement de l'insertion et pour se déclipser dès que le piston rencontre une force F2(t) de sens inverse et supérieure à une intensité prédéterminée (une valeur seuil non nulle).

F1(t) et F2(t) sont de sens opposé, idéalement de même direction (mais pas obligatoirement) et au moins temporairement non nul. Dans certains modes de réalisation, F1(t) et F2(t) peuvent s'exercer temporairement simultanément sur ledit piston. Dans un autre mode de réalisation, les moyens de propulsion n'exercent plus de force mais grâce à l'énergie cinétique du propulseur et F2(t) exercée contre le piston, les moyens de retenue libèrent le piston. Ainsi, lesdits moyens de retenue se désolidarisent permettant au propulseur de continuer sa course (son déplacement) sans exercer de force ou de contrainte sur le piston ou de façon limitée.

Dit autrement, lors du déclenchement du dispositif, le propulseur entraine le piston en direction du tissu en exerçant sur lui une force F1(t). Dès que le piston entre en contact avec un élément (par exemple le tissu ciblé, une butée, ), cet élément va générer une contre force (nommée F2(t)) - dans le sens opposé de l'insertion - qui s'appliquera sur l'ensemble aiguille-piston (appelé également piston). F2(t) va graduellement ou instantanément croître tant que le piston continue sa course mis en mouvement par le propulseur, jusqu'à atteindre une valeur prédéterminée dont l'intensité est telle que les moyens de retenue sont contraints de libérer (par exemple par déclipsage) le piston des moyens de propulsion.

Dans une réalisation possible, les moyens de propulsion sont constitués d'un propulseur logeant un ressort ou un élastique ou une lame élastique.

Les moyens de retenue peuvent être façonnés et agencés en fonction des caractéristiques des moyens de propulsion et des caractéristiques du tissu dans lequel les aiguilles doivent être insérées.

Dans un exemple ne faisant pas partie de l'invention, moyens de retenue se désolidarisent à cause de l'impact ou par d'autres moyens tels qu'une butée ou de façon manuelle afin que le propulseur n'exerce plus de pression sur l'ensemble aiguille-piston au moins avant le début de l'injection de la solution.

Comme décrit plus haut, suite à l'impact des aiguilles contre le tissu et/ou à la force F2(t) après libération des moyens de retenue, l'ensemble aiguille-piston peut être projeté dans le sens opposé de l'insertion et causer ainsi le retrait partiel ou total des aiguilles bien que ces dernières aient été initialement correctement insérées. De plus, durant l'injection, selon les caractéristiques des micro-aiguilles (par exemple la direction du ou des canaux et/ou la forme des aiguilles décrit par les demandes de brevets WO 2011/006699, WO 2003/015860, WO 2006/025786, EP1669100 les aiguilles peuvent sortir partiellement ou totalement de leurs logements. Ce phénomène peut être, par exemple, dû au principe d'action-réaction : l'action créée par la force du liquide injecté dans le tissu génère, par réaction une force de sens opposée capable de déloger les aiguilles du tissu. Afin d'assurer la bonne insertion des aiguilles et de limiter les fuites de la solution à injecter, les moyens de pression permettent aux aiguilles de rester insérées dans le tissu après l'insertion et pendant l'injection.

Ainsi, dans le cadre de la présente invention, au moins avant l'injection, une pression de l'ensemble aiguille-piston contre le tissu est préférablement exercée par des moyens de pression. Toutefois, pour une injection optimale de la solution dans ou à travers le tissu, ces moyens de pression doivent avoir une force suffisamment faible du début à la fin de la formation de la papule afin de ne pas limiter la formation de cette papule au cours de l'injection.

Exprimé différemment, lesdits moyens de pression exercent une force F3(t) selon une direction correspondant à l'orientation principale dans le sens de l'insertion (même sens que F1(t)), c'est-à-dire en direction du tissu, sans pour autant gêner la formation de la papule.

Cette force F3(t) peut être générée par un seul élément ou plusieurs éléments qui peuvent être, par exemple et de manière non exhaustive, une tubulure, un ressort, un élastique, une cartouche de gaz, de l'air comprimé, une force électromagnétique, la génération de gaz par réaction chimique entre au moins deux composés, une lame élastique et/ou le poids de l'ensemble piston-aiguille.

Dans le présent document, les forces de frottement peuvent être considérées négligeables (ou du moins très faibles comparées aux autres forces en jeu) et les forces F1(t), F2(t), F3(t) sont au moins temporairement non nulles. Dans un mode réalisation, le valeur du seuil au-dessus duquel les moyens de retenue libèrent le piston est comprise entre 0 et 10 N, idéalement entre 0.5 et 5 N. La force F3(t) est comprise entre 0 et 10N, idéalement inférieure à 5N.

Dans un mode de réalisation, la formation de la papule va générer une force F4(t) contre le piston dans le même sens que F2(t). Dans un mode de réalisation, F4(t) est supérieur à F3(t) et entraine un recul du piston par rapport à sa position avant l'injection.

Dans un autre mode de réalisation, les moyens de pression exercent une force dès l'activation des moyens de propulsion. Le ressort des moyens de propulsion peut ainsi se coupler aux moyens de pression pour former la source d'énergie nécessaire pour les moyens de propulsion.

Dans un autre mode de réalisation, les moyens de propulsion sont eux-mêmes responsables de la force résiduelle en fin de course. Cela peut, par exemple, correspondre à la fin de course d'un ressort ou d'un élastique utilisé pour la propulsion du piston, le dit ressort ou élastique étant calculé de manière à ce que cette force résiduelle en fin de course corresponde à la force résiduelle nécessaire.

Dans un autre mode de réalisation, les moyens de pression peuvent également servir à propulser la solution dès que les micro-aiguilles sont logées dans le tissu du patient et que les moyens de rétention sont libérés, réalisant ainsi un auto-injecteur. Dans un autre mode de réalisation, après libération des moyens de retenue les moyens de propulsion permettent d'injecter la solution dans le tissu.

De préférence, les moyens de propulsion exercent une force dans le sens correspondant à l'insertion et d'une valeur supérieure ou égale aux moyens de pression.

Dans un mode de réalisation préféré, le dispositif est façonné de manière à ce que tout appui du dispositif contre le tissu n'exerce pas - ou de façon marginale - de pression sur l'ensemble aiguille-piston de sorte que la formation de la papule ne puisse être gênée. L'extrémité distale du dispositif est composée d'une zone de contact en forme de bague ou de pieds stabilisateurs permettant de positionner le dispositif sur le tissu selon un angle désiré et de façon suffisamment éloigné n'influant pas, ou peu, sur les caractéristiques mécaniques du tissu. De plus, ladite bague ou pied stabilisateur n'ont ici aucun effet sur la profondeur d'insertion des aiguilles.

Dans un mode de réalisation, le piston est initialement dans une position 0 (ou suite à l'armement des moyens de propulsion), où l'ensemble aiguille-piston est logé de façon sécurisé dans le boitier du dispositif. Le dispositif est configuré de manière à ce que le piston atteigne une première position, consécutivement à l'activation des moyens de propulsion, formant une distance D1 entre la face distale du piston et l'extrémité proximale du boitier; puis une deuxième position avant l'injection, formant une distance D2 entre la face distale du piston et l'extrémité proximale du boitier; puis une troisième position, consécutivement à l'injection d'au moins une solution dans ou à travers le tissu, formant une distance D3 entre la face distale du piston et l'extrémité proximale du boitier; D3 étant inférieure ou égale à D1 et à D2. Les moyens de pression sont destinés à maintenir ladite aiguille au moins partiellement insérée dans le tissu et à autoriser au moins un recul maîtrisé du piston lors du passage de la première à la deuxième position puis jusqu' à la troisième position.

Le dispositif selon l'invention peut permettre, selon les caractéristiques du tissu et/ou la manipulation par l'opérateur et/ou des caractéristiques du dispositif, d'avoir D1 supérieure à D2 ou inversement D1 inférieure ou égale à D2

De préférence, le dispositif dispose d'une butée limitant la course du piston. Cette butée peut être positionnée de sorte que le piston puisse sortir en sailli de l'extrémité distale du dispositif. Cette butée peut également permettre de désolidariser le piston du propulseur.

Dans un mode de réalisation, le dispositif dispose d'une butée limitant la course du propulseur, de sorte que le propulseur ne puisse exercer de pression sur le piston durant l'injection.

Dans un mode de réalisation, l'injection peut être déclenchée automatiquement ou manuellement une fois la ou les aiguilles insérées dans le tissu, mais préférentiellement elle commencera une fois que le piston soit revenu en deuxième position, c'est-à-dire lorsque le propulseur n'exerce plus de force contre le tissu.

Dans un mode de réalisation préférée, une seringue ou un réservoir est en connexion fluidique avec les aiguilles.

De préférence, le propulseur est mis en mouvement par le relâchement d'une énergie potentielle qui peut être déclenché à l'aide des moyens d'activation , par exemple un bouton ou autres mécanismes. Cette énergie peut être stockée sous des formes variées : par exemple, mais de manière non exhaustive, ressort, ressort à lamelle, cartouche de gaz, air comprimé, force électromagnétique, génération de gaz par réaction chimique entre au moins deux composés.

Dans un mode de réalisation possible, la source d'énergie peut être l'opérateur lui-même. En appuyant avec une force suffisante, il générera par un mécanisme interne au dispositif la vitesse nécessaire à la pénétration de la ou des aiguilles dans le tissu.

Le dispositif peut comprendre également un mécanisme de sécurité, permettant de verrouiller les moyens d'activation afin d'éviter un déclenchement involontaire. Le mécanisme de sécurité peut se présenter sous différentes formes, l'appui de la bague ou du dispositif sur le tissu peut également déverrouiller la détente et permettre le déclenchement.

Le dispositif peut comprendre aussi un récipient utilisé pour stocker la solution à administrer. Ce récipient peut être positionné sur le corps du dispositif ou être directement intégré au dispositif ou être simplement en connexion fluidique avec les aiguilles. L'activation de ce récipient, qui provoquera l'injection de la substance dans le tissu, peut être soit manuelle, soit automatique grâce à l'aide par exemple des moyens de pression, des moyens de propulsion, de l'apesanteur ou tout autres éléments ou mécanismes.

Dans ce deuxième cas, l'activation du dispositif entraînera, une fois les aiguilles en place, l'activation du récipient. Ce récipient peut prendre diverses formes telles que - et ceci de façon non exhaustive - un réservoir semi rigide, une poche souple, une seringue, une carpule.

Dans un mode de réalisation possible, avant l'injection et avant l'activation des moyens de propulsion, l'opérateur peut vider l'air éventuellement contenu dans la connexion fluidique ou le récipient. Le dispositif peut comprendre une fenêtre à hauteur de l'aiguille, permettant ainsi de voir la première goûte, preuve que la connexion fluidique est vide d'air.

Le dispositif peut comprendre un mécanisme ou un élément qui rend difficilement accessible l'aiguille de manière à prévenir les blessures, avant et/ou après l'insertion ainsi qu'après l'injection. Cette protection peut s'obtenir notamment en rétractant l'aiguille dans le corps du dispositif, ou dans le support lui-même, ou en coulissant ou en emboitant un cache, ou le déploiement d'un élément de protection qui empêche l'accès par inadvertance à l'aiguille au moins après le retrait du dispositif du tissu cible. Un tel système de protection peut se déclencher automatiquement dès le retrait des micro-aiguilles du tissu du patient, par exemple du fait de la force résiduelle.

Dans une réalisation possible, les aiguilles du dispositif sont des micro-aiguilles. Par micro-aiguilles, on entend des aiguilles dont les dimensions sont adaptées pour cibler de préférence le territoire intradermique. Cette zone a une épaisseur variable selon le patient ainsi que selon l'emplacement sur le corps d'un même patient. Elle est de l'ordre de quelques centaines de microns à quelques millimètres. La micro-aiguille pourra cependant être un peu plus longue que l'épaisseur maximum de cette zone pour tenir compte de la pénétration des micro aiguilles dans le tissu qui pourrait n'être que partielle.

Le dispositif décrit par le présent document peut également permettre le prélèvement de solution.

### Liste des figures

L'invention sera mieux comprise ci-après au moyen de quelques exemples illustrés. Il va de soi que l'invention ne se limite pas à ces modes de réalisation.
Figure 1 : Vue d'ensemble de l'inserteur.
Figures 2 : Vue éclatée de l'inserteur et vue détaillée de l'ensemble piston-propulseur.
Figure 3 : Vue de coupe de l'inserteur avec et sans ressort (4a) utilisé comme moyen de pression.
Figure 4 : Inserteur chargé et prêt à être actionné.
Figures 5 : Inserteur en position 1.
Figures 6 : Inserteur en position 2.
Figures 7 : Inserteur en position 3.
Figures 8 : Lancement de l'ensemble Piston-Propulseur.
Figure 9 : Début d'insertion de l'ensemble Piston-Propulseur.
Figure 10 : Ensemble Piston-Propulseur en position 1.
Figure 11 : Ensemble Piston-Propulseur en position 2.
Figure 12 : Ensemble Piston-Propulseur en position 3.

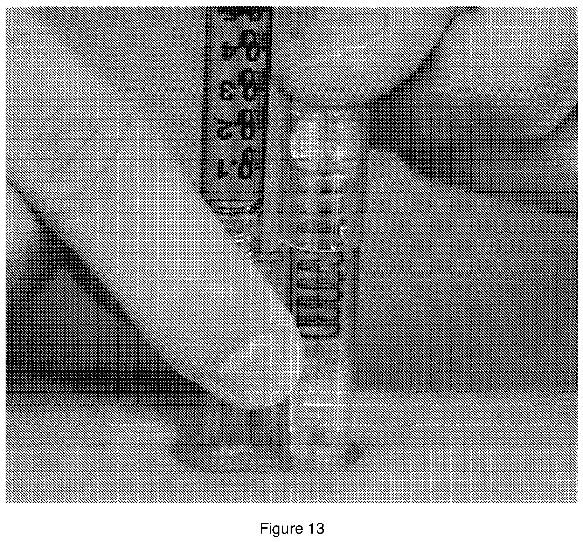

Figure 13 : Inserteur en après actionnement du propulseur.

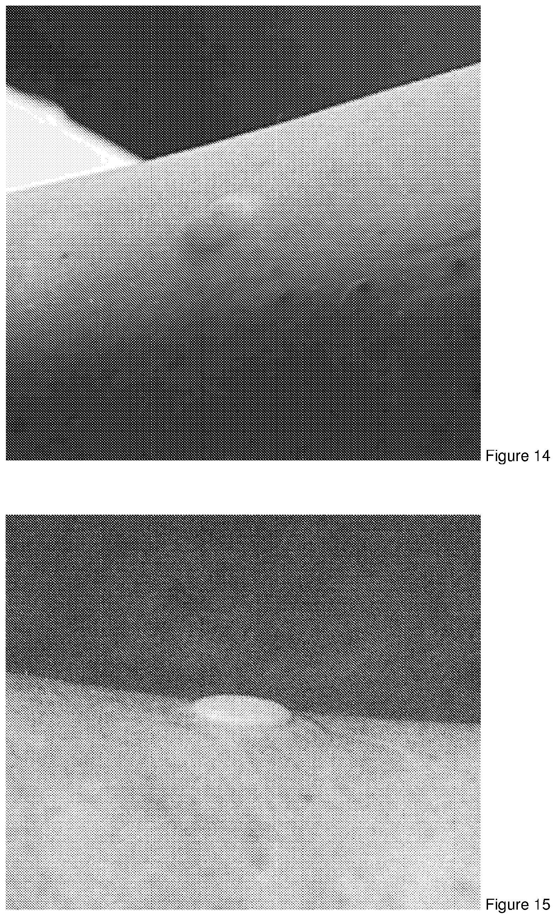

Figure 14 : Papule formée après injection de 0.2 ml de solution.
Figure 15 : Papule formée après injection de 0.5 ml de solution.

### Description détaillée de l'invention

Dans le présent document, la description détaillée de l'invention comporte des modes de réalisation de dispositifs, de systèmes et de méthodes présentés à titre d'illustration. Il est bien entendu que d'autres modes de réalisation sont envisageables et peuvent être apportées tout en restant dans la portée des revendications décrites en annexe. La description détaillée qui suit, par conséquent, ne doit pas être prise dans un sens limitatif.

Sauf indication contraires, les termes scientifiques et techniques utilisé dans le présent document ont des significations couramment utilisés par l'homme du métier. Les définitions apportées dans ce document sont mentionnées afin de faciliter la compréhension des termes fréquemment utilisés et ne sont pas destinées à limiter la portée de l'invention.

Les indications de direction utilisées dans la description et les revendications, telles que "haut", "bas", "gauche", "droite", "supérieur", "inférieur", et autres directions ou orientations sont mentionnées afin d'apporter plus de clarté en référence aux figures. Ces indications ne sont pas destinées à limiter la portée de l'invention.

Les verbes « avoir », « comprendre », « inclure » ou équivalent sont utilisés, dans le présent document, dans un sens large et signifie de façon générale « inclut, mais sans s'y limiter »

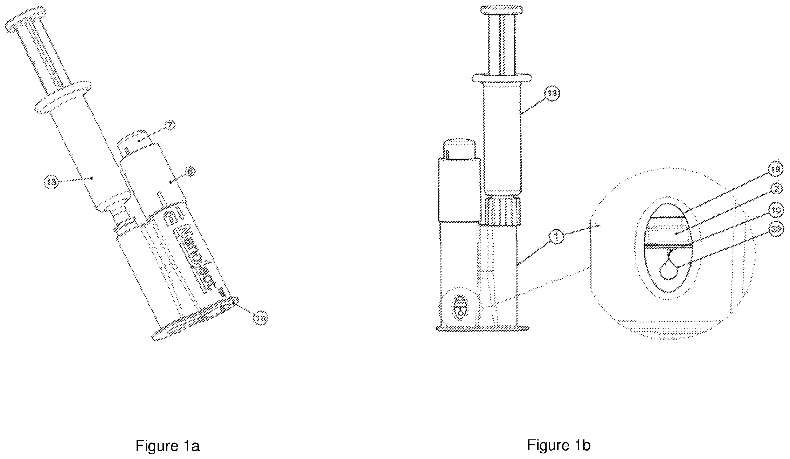

Les figures 1a et 1b représentent les différents composants du dispositif complet vu de l'extérieur avant l'insertion : le boitier (1), la face distale du dispositif (1a), le déclencheur (7) et l'élément de sécurité du déclencheur (6).

Afin de faire le vide d'air de la connexion fluidique entre la seringue (13) et l'aiguille (10) avant l'injection, le dispositif dispose d'une fenêtre (19) pour permettre de voir l'aiguille (10) et de voir la première goute (20) de la solution avant d'effectuer l'insertion.

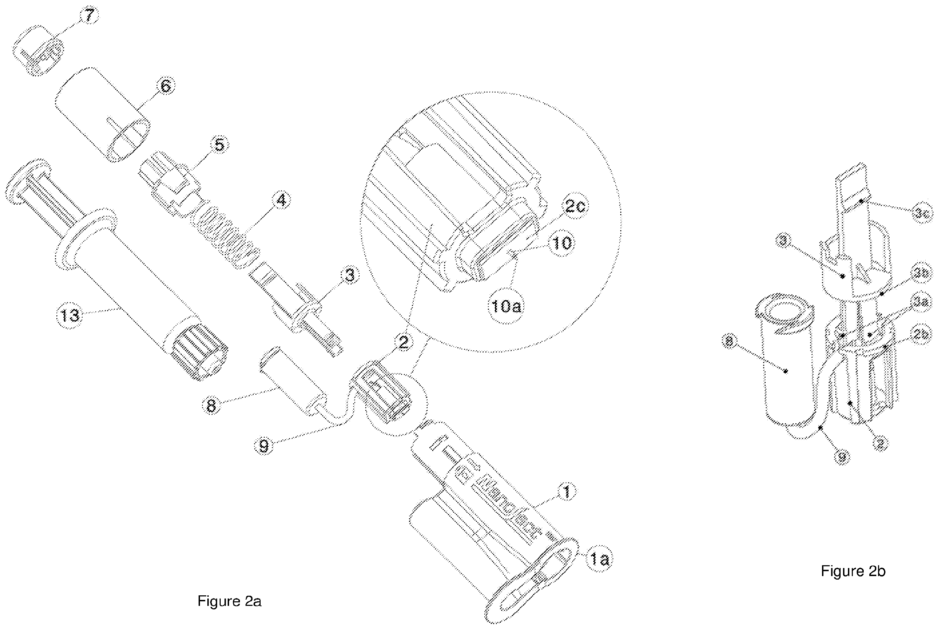

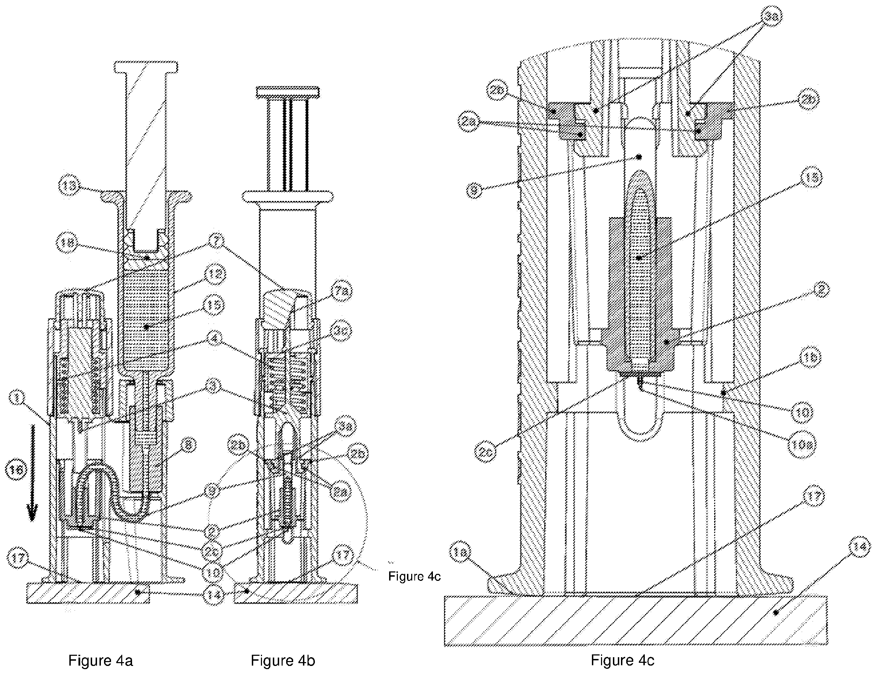

La figure 2a est une vue en éclaté du dispositif qui divulgue les différents éléments présents à l'intérieur :
- Le lueur (8), qui permet la connexion avec la seringue (13), est en connexion fluidique avec l'aguille (10) grâce à la tubulure (9).
- Les moyens de propulsion sont constitués d'un ressort (4), qui est la principale source d'énergie pour l'insertion, comprimé entre un élément solidaire au boitier (5) et le propulseur (3) monté mobile dans le boitier. Une telle disposition assure un mouvement guidé par la paroi intérieure du boîtier selon une direction correspondant à l'orientation principale (16 représenté sur la figure 4).
- L'aiguille (10) est solidaire du piston (2) et fait saillie par rapport à la face distale du piston (2c). L'aiguille (10) dispose elle-même d'une extrémité distale (10a) pointue. La taille de l'aiguille est fonction des caractéristiques du tissu où doit être effectuée l'injection. La distance entre la face distale (10a) de l'aiguille et celle (2c) du piston représente la profondeur à laquelle doit être effectué l'injection. La taille de la face distale (2c) permet de garantir cette profondeur maximum.

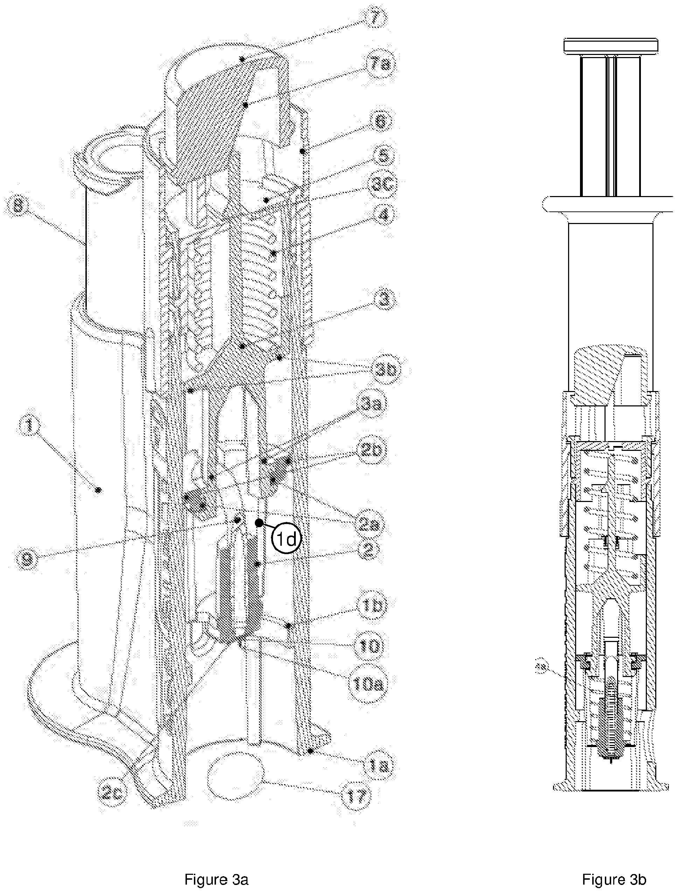

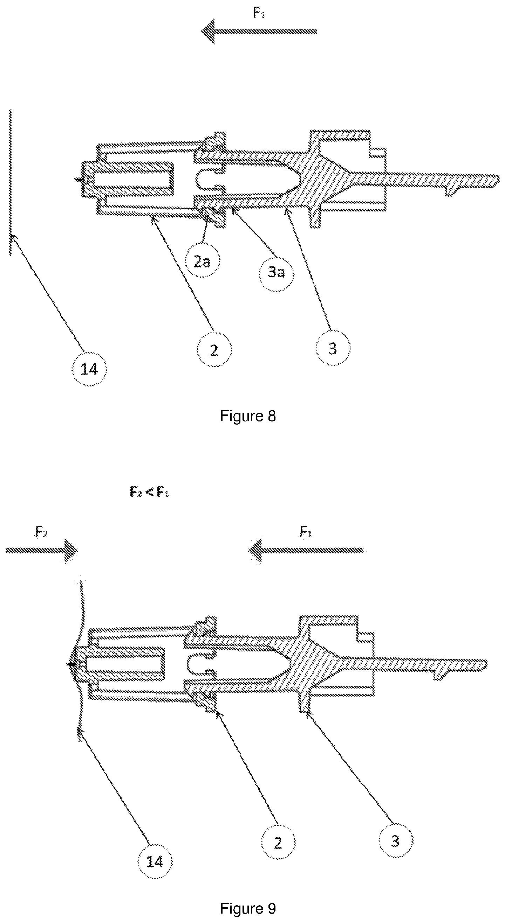

La figure 2b présente l'ensemble piston-propulseur (2, 3). Le propulseur dispose de moyens de retenue (3a) permettant de rendre temporairement solidaire cet ensemble piston-propulseur (2, 3). Le piston est façonné de manière à recevoir les moyens de retenue (3a) grâce aux éléments de réception (2a représenté sur la figure 3a). La figure 8 permet de voir avec plus de précision comment le piston est rendu solidaire au propulseur. Les moyens de retenue (3a) disposent de deux extrusions (par moyens de retenue) chanfreinés afin de clipser le piston (2) au propulseur (3) lors du montage de l'ensemble et de déclipser avant l'injection.

Les figures 3a et 3b représentent l'inserteur chargé, c'est-à-dire ayant le ressort (4) comprimé et prêt à effectuer l'insertion. La figure 3b se distingue de la figure 3a uniquement grâce à la présence du ressort (4a) qui agit comme moyens de pression.

Selon la figure 3a, l'aiguille (10) est solidairement fixée au piston (2) lui-même solidaire du propulseur (3). Le ressort (4) est comprimé entre le propulseur (3) et un élément solidaire du boitier (5). Cette position appelée « dispositif chargé » est maintenu temporairement par l'élément de retenue (3c) à l'élément solidaire du boitier (5). L'opérateur doit déverrouiller l'élément de sécurité (6) pour enfoncer le déclencheur (7) qui, à son tour, libère l'élément de retenue (3c).

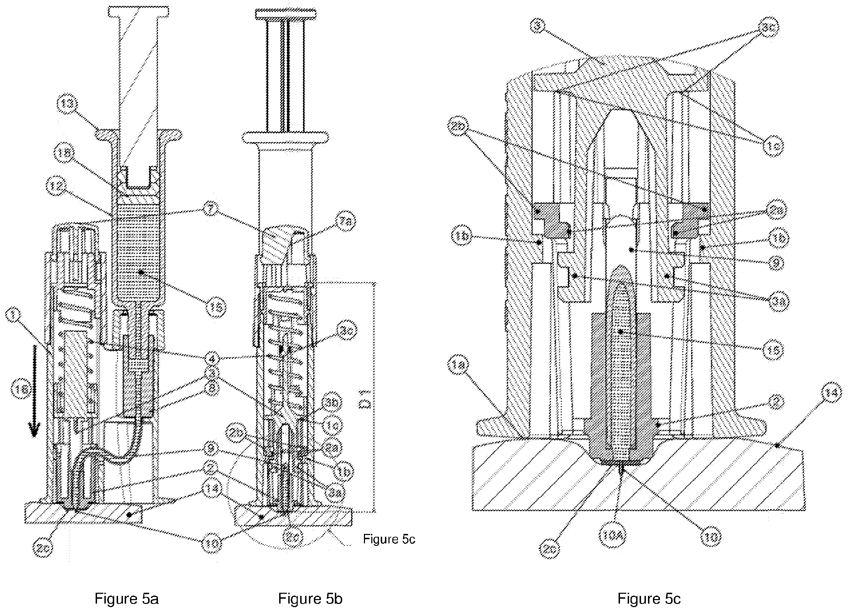

Le boitier dispose de moyens de guidage (1d) contraignant le piston à un mouvement selon la direction principale afin de venir en contact sur une zone prédéfinie (17). Le boitier contient également des butées (1b) et (1c représenté sur la figure 5) limitant respectivement la course du piston et du propulseur. La butée (1b) permet néanmoins à ce que la face distale (2c) du piston dépasse l'extrémité distale (1a) du dispositif et peut contraindre les éléments de retenue (3c) à se déclipser si la résistance du tissu n'a permis ce déclipsage avant. La butée (1c) maintient le propulseur (3) afin qu'il n'exerce plus de pression via son ressort (4) directement sur le piston (2).

Les figures 4 à 7 divulguent le dispositif complet du positionnement à l'injection.

Les figures 4a, 4b et 4c représentent le dispositif positionné sur le tissu afin d'injecter la solution sous la zone de contact (17). L'ensemble piston-propulseur (2, 3) est solidaire, le ressort (4) compressé. A ce stade, l'opérateur peut exercer une légère pression sur le piston (18) de la seringue afin de faire le vide d'air de la connexion fluidique comprenant le lueur (8) et la tubulure (9). Une fenêtre (19 représenté sur la figure 1b) permet de vérifier lorsque la première goute (20) de la solution (15) sort de l'aiguille (10).

Les figures 5a, 5b et 5c représentent le dispositif avec le piston (2) en position 1 formant ainsi une distance D1 entre l'extrémité proximale du boitier et la face distale (2a) du piston. Selon cette configuration, le piston a ainsi atteint sa position la plus éloigné de l'extrémité proximale du boitier. Il est toutefois possible que l'opérateur exerce une telle pression sur le dispositif qu'un bourrelet artificielle se forme. Dans ce cas, D1 pourrait ne pas être la distance maximale entre l'extrémité proximale du boitier et la face distale (2a) du piston.

A ce stade, les moyens de retenue (3a) peuvent se déclipser libérant ainsi le piston (2) de toute force exercée directement par le propulseur (3). Le déclipsage peut être causé par la résistance élastique du tissu ou par la butée 1b, tous deux exerçant une force s'opposant à la force exercée par le propulseur. Toutefois, l'ensemble aiguille-piston (10, 2) est contraint par des moyens de pression (par exemple 4a représenté sur la figure 3b) exerçant une force dans le sens de l'insertion (16) afin de maintenir l'aiguille (10) correctement insérée dans le tissu (14). A noter que la forme en S couché de la tubulure (9) peut également définir les moyens de pression. Les moyens de pression peuvent être actif dès l'activation du dispositif ou au moins dès que la face distale (10a) de l'aiguille touche le tissu (14).

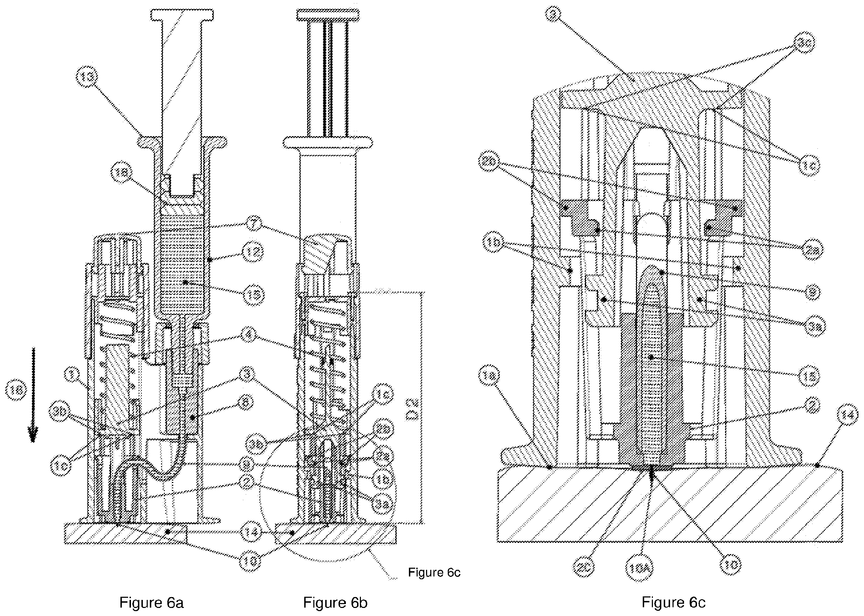

Les figures 6a, 6b et 6c représentent le dispositif avec le piston (2) en position 2 formant ainsi une distance D2 entre l'extrémité proximale du boitier et la face distale (2a) du piston. Cette position correspond normalement à un retour à l'équilibre où seuls les moyens de pression du dispositif exercent une force dans le sens de l'insertion (16).

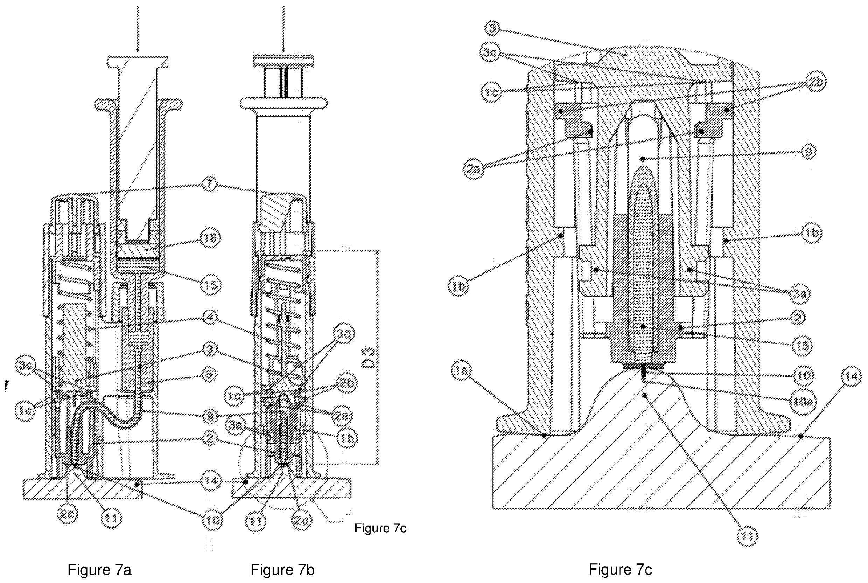

Les figures 7a, 7b et 7c représentent le dispositif avec le piston (2) en position 3 formant ainsi une distance D3 entre l'extrémité proximale du boitier et la face distale (2a) du piston. Cette distance D3 est inférieure à D1 et/ou D2. A ce stade, l'opérateur exerce une pression sur le piston (18) de la seringue (13), injectant la solution (15) sous la zone de contact (17). L'injection a pour conséquence une accumulation de la solution (15) sous la zone de contact (17) déformant cette dernière et formant ainsi une papule (11). Les moyens de pression sont configurés pour exercer une pression minimale afin de ne pas s'opposer à la formation de la papule (11). L'ensemble aiguille-piston effectue ainsi un recul qui est maîtrisée grâce aux moyens de pression.

Les figures 8 à 12 divulguent l'interaction entre le piston (2) et le propulseur (3) du positionnement à l'injection.

La figure 8 présente le lancement de l'ensemble piston-propulseur solidaire grâce aux moyens de retenue (3a). Le lancement du propulseur génère une force F1 exercée par les moyens de propulsion et entraîne le piston (2) en direction du tissu.

La figure 9 présente l'aiguille qui commence à pénétrer le tissu. L'ensemble piston-propulseur est encore solidaire et exerce une force F1 contre le tissu. Les caractéristiques mécaniques du tissu engendrent une force de sens opposé appelée F2. Plus le piston avance contre le tissu plus la force F2 augmente.

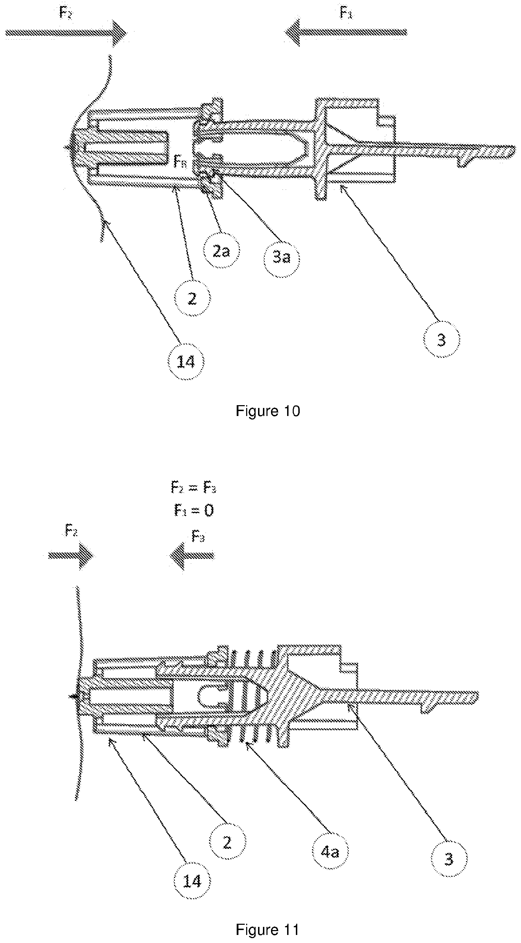

La figure 10 présente le piston en position 1. L'aiguille a parfaitement pénétré le tissu et la force F2 a atteint une valeur prédéterminée qui permet de déclipser les moyens de retenue. Exprimé différemment, les sens opposés des forces F2 et F1 induisent une force radiale Fr, dirigée selon une direction perpendiculaire par rapport à la direction des forces F1 et F2. Les forces F1, F2 et Fr font glisser le propulseur à l'intérieur du piston et rendre les moyens de retenue inopérants.

La figure 11 présente le piston en position 2 où F1 devient nulle car le piston est déconnecté du propulseur. Une nouvelle force appelée F3 est générée par les moyens de pressions (4a). F3 est égale à F2 ce qui correspond à un état d'équilibre. L'aiguille est insérée dans le tissu et les moyens de pressions (4a) maintiennent l'aiguille correctement insérée.

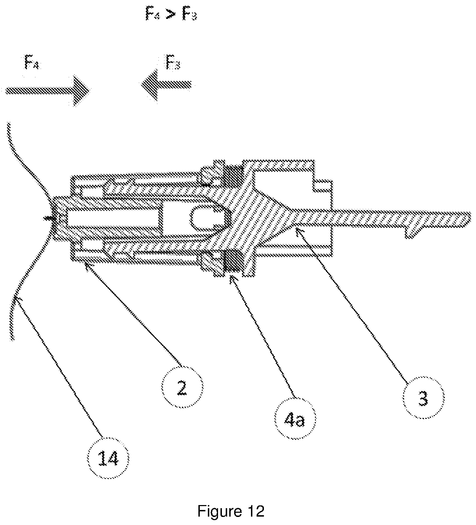

La figure 12 présente le piston en position 3. L'injection de la solution cause la formation d'une papule. Les moyens de pression exercent une force F3 inférieure à la force F4 générée par la formation de la papule. La force F3 permet de maintenir l'aiguille correctement insérée durant l'injection.

### Références numériques utilisées dans les figures

- 1.: Boitier
- 1a: Face distale du dispositif
- 1b: Elément de butée pour le piston
- 1c: Elément de butée pour le propulseur
- 1d: Moyens de guidage
- 2.: Piston
- 2a: Eléments de réception des moyens de retenue du propulseur
- 2b: Eléments de retenue limitant la course du piston
- 2c: Face distale du piston
- 3.: Propulseur
- 3a: Moyens de retenue du propulseur au piston
- 3b: Eléments de retenue limitant la course du propulseur
- 3c: Moyens de retenue du propulseur au boitier
- 4.: Ressort du moyen de propulsion
- 4a: Ressort du moyen de pression
- 5.: Elément solidaire du boitier
- 6.: Elément de sécurité du déclencheur
- 7.: Déclencheur
- 7a: Elément de déclenchement
- 8.: Luer
- 9.: Tubulure
- 10.: Aiguille
- 10a: Extrémité distale de l'aiguille
- 11.: Papule
- 12.: Réservoir
- 13.: Seringue
- 14.: Tissu
- 15.: Solution
- 16.: Sens de l'insertion
- 17.: Zone de contact
- 18.: Piston de la seringue
- 19.: Fenêtre
- 20.: Goute

## Revendications

1. Dispositif pour l'insertion d'au moins une aiguille creuse pour l'injection ou le prélèvement d'une solution dans un tissu, ledit dispositif comprenant :
a. un boitier (1) défini par une extrémité distale (1a) destinée à venir en contact avec le tissu et une extrémité proximale opposée,
b. un piston (2) monté mobile à l'intérieur du boitier (1) comprenant :
i. une extrémité distale (2c) destinée à venir à proximité du ou en contact avec le tissu,
ii. ladite au moins une aiguille creuse (10) destinée à pénétrer le tissu,
c. des moyens de propulsion (3, 4) adaptés pour entrainer le piston (2) en direction de l'extrémité distale (1a) du dispositif en appliquant sur lui une force F1(t) ;
le dispositif étant **caractérisé**
**en ce que** lesdits moyens de propulsion (3, 4) et/ou ledit piston (2) comprennent des moyens de retenue (3a) pour rendre temporairement solidaires les moyens de propulsion (3, 4) dudit piston (2) pendant que ce dernier est mis en mouvement par les moyens de propulsion (3, 4), et
**en ce que** lesdits moyens de retenue (3a) sont conçus pour désolidariser le piston du moyen de propulsion permettant aux moyens de propulsion de continuer leur course sans exercer de force ou de contrainte sur le piston ou de façon limitée) lorsqu'une force F2(t), de valeur déterminée, supérieure à une valeur seuil non nulle et de sens opposé à F1(t), s'exerce sur le piston (2).

2. Le dispositif selon la revendication 1, dans lequel le dispositf comprend en outre des moyens de pression destinés à maintenir ladite aiguille au moins partiellement insérée dans le tissu et à autoriser au moins un recul maîtrisé du piston (2) au moins durant l'injection de la solution ; lesdits moyens de pression exerçant une force F3(t) de même sens que F1(t).

3. Dispositif selon la revendication 1, agencé de manière à ce que les forces F1(t) et F2(t) s'exercent au moins temporairement simultanément contre le piston (2).

4. Dispositif selon la revendication 2, adapté de manière à ce que F3(t) soit inférieure à une force maximale F4(t) exercée contre le piston (2), F4(t) étant induite par la création de la papule consécutivement à l'injection de ladite solution (15).

5. Dispositif selon l'une quelconque des revendications 2 ou 4, dans lequel lesdits moyens de pression sont adaptés de manière à exercer la force F3(t) au moins avant l'injection de la solution dans le tissu.

6. Dispositif selon l'une quelconque des revendications précédentes, dans lequel lesdits moyens de pression sont adaptés de manière à s'activer automatiquement ou manuellement.

7. Dispositif selon l'une quelconque des revendications précédentes, dans lequel les moyens de propulsion (3, 4) sont adaptés pour exercer une force d'une valeur supérieure ou égale aux moyens de pression.

8. Dispositif selon l'une quelconque des revendications 2 ou 4. dans lequel F3(t) est générée uniquement ou cumulativement par une tubulure (9), un ressort (4a), un élastique, une cartouche de gaz, de l'air comprimé, une force électromagnétique, la génération de gaz par réaction chimique entre au moins deux composés, une lame élastique, le poids de l'ensemble piston-aiguille (2, 10) et/ou induite par le frottement du piston contre les parois internes du boitier (1).

9. Dispositif selon l'une quelconque des revendications précédentes, comprenant un réservoir en communication fluidique avec au moins une aiguille (10).

10. Dispositif selon l'une quelconque des revendications précédentes, comprenant une seringue (13) comportant un piston (18) destiné à expulser à travers l'aiguille (10) une solution (15).

11. Dispositif selon la revendication 10, dans lequel la seringue (13) est orientée selon une direction identique ou distincte de celle définie par le déplacement de l'aiguille (10).

12. Dispositif selon l'une quelconque des revendications précédentes, dans lequel la solution contenu dans le réservoir ou la seringue est injecté dans ou à travers le tissu de façon manuelle ou automatique au moins après libération du piston des moyens de propulsion.

13. Dispositif selon l'une quelconque des revendications précédentes, comprenant une tubulure (9) assurant une communication fluidique entre un réservoir et l'aiguille (10).

14. Dispositif selon l'une quelconque des revendications précédentes, dans lequel le boitier (1) comporte un premier élément de butée (1b) disposé de manière à retenir le piston (2) lorsqu'il est entraîné vers ladite extrémité distale.

15. Dispositif selon la revendication 14, dans lequel le boitier comporte un deuxième élément de butée (1c) disposé de manière à retenir le propulseur (3) lorsqu'il est entraîné vers ladite extrémité distale (1a).

16. Dispositif selon la revendication 1, comprenant des moyens de protection activable manuellement ou automatiquement après l'injection, de sorte que ladite au moins une aiguille (10) soit protégée au moins après le retrait du dispositif du tissu cible.

## Patentansprüche

1. Vorrichtung zum Einführen zumindest einer Hohlnadel zum Injizieren oder Entnehmen einer Lösung in ein bzw. aus einem Gewebe, wobei die Vorrichtung Folgendes umfasst:
a. ein Gehäuse (1), das durch ein distales Ende (1a), das dazu bestimmt ist, mit dem Gewebe in Kontakt zu kommen, und ein gegenüberliegendes proximales Ende definiert ist,
b. einen Kolben (2), der beweglich innerhalb des Gehäuses (1) montiert ist, umfassend:
i. ein distales Ende (2c), das dazu bestimmt ist, in die Nähe des Gewebes oder mit diesem in Kontakt zu kommen,
ii. die zumindest eine Hohlnadel (10), die dazu bestimmt ist, in das Gewebe einzudringen,
c. Vortriebsmittel (3, 4), die dazu ausgelegt sind, den Kolben (2) durch Aufbringen einer Kraft F1(t) auf denselben in Richtung des distalen Endes (1a) der Vorrichtung zu bewegen;
wobei die Vorrichtung **dadurch gekennzeichnet ist,**
**dass** die Vortriebsmittel (3, 4) und/oder der Kolben (2) Haltemittel (3a) umfassen, um die Vortriebsmittel (3, 4) vorübergebend fest mit dem Kolben (2) zu verbinden, während dieser durch die Vortriebsmittel (3, 4) in Bewegung gesetzt wird, und
**dass** die Haltemittel (3a) dazu gestaltet sind, den Kolben von den Vortriebmitteln zu lösen, damit die Vortriebsmittel ihren Weg fortsetzen können, ohne Kraft oder Zwang auf den Kolben auszuüben oder dies in eingeschränkter Weise zu tun, wenn eine Kraft F2(t) mit einem bestimmten Wert, der größer als ein Schwellenwert ungleich Null ist, die in zu F1 (t) entgegengesetzter Richtung wirkt, auf den Kolben (2) ausgeübt wird.

2. Vorrichtung nach Anspruch 1, wobei die Vorrichtung ferner Druckmittel umfasst, die dazu bestimmt sind, die Nadel zumindest teilweise in das Gewebe eingeführt zu halten und zumindest ein kontrolliertes Zurückziehen des Kolbens (2) zumindest während des Injizierens der Lösung zu ermöglichen; wobei die Druckmittel eine Kraft F3(t) ausüben, die in derselben Richtung wie F1 (t) wirkt.

3. Vorrichtung nach Anspruch 1, die so angeordnet ist, dass die Kräfte F1(t) und F2(t) zumindest zeitweise gleichzeitig gegen den Kolben (2) ausgeübt werden.

4. Vorrichtung nach Anspruch 2, die so ausgelegt ist, dass F3(t) kleiner als eine gegen den Kolben (2) ausgeübte maximale Kraft F4(t) ist, wobei F4(t) durch die Bildung der Papel nach der Injektion der Lösung (15) ausgelöst wird.

5. Vorrichtung nach einem der Ansprüche 2 oder 4, wobei die Druckmittel so ausgelegt sind, dass sie die Kraft F3(t) zumindest vor dem Injizieren der Lösung in das Gewebe ausüben.

6. Vorrichtung nach einem der vorangehenden Ansprüche, wobei die Druckmittel so ausgelegt sind, dass sie automatisch oder manuell aktiviert werden.

7. Vorrichtung nach einem der vorangehenden Ansprüche, wobei die Vortriebsmittel (3, 4) so ausgelegt sind, dass sie eine Kraft ausüben, die größer als oder so groß wie die der Druckmittel ist.

8. Vorrichtung nach einem der Ansprüche 2 oder 4, wobei F3(t) allein oder kumulativ durch einen Stutzen (9), eine Feder (4a), ein Gummiband, eine Gaskartusche, Druckluft, eine elektromagnetische Kraft, die Erzeugung von Gas durch chemische Reaktion zwischen zumindest zwei Verbindungen, eine elastische Klinge, das Gewicht der Kolben-Nadel-Einheit (2, 10) erzeugt und/oder durch die Reibung des Kolbens an den Innenwänden des Gehäuses (1) verursacht wird.

9. Vorrichtung nach einem der vorangehenden Ansprüche, umfassend einen Behälter, der mit zumindest einer Nadel (10) in Fluidverbindung steht.

10. Vorrichtung nach einem der vorangehenden Ansprüche, umfassend eine Spritze (13), die einen Kolben (18) aufweist, der dazu bestimmt ist, eine Lösung (15) durch die Nadel (10) auszustoßen.

11. Vorrichtung nach Anspruch 10, wobei die Spritze (13) in einer Richtung ausgerichtet ist, die gleich der durch die Bewegung der Nadel (10) definierten Richtung ist oder sich von dieser unterscheidet.

12. Vorrichtung nach einem der vorangehenden Ansprüche, wobei die im Behälter oder in der Spritze enthaltene Lösung zumindest nach der Freigabe des Kolbens der Vortriebsmittel manuell oder automatisch in oder durch das Gewebe injiziert wird.

13. Vorrichtung nach einem der vorangehenden Ansprüche, umfassend einen Stutzen (9), der eine Fluidverbindung zwischen einem Behälter und der Nadel (10) gewährleistet.

14. Vorrichtung nach einem der vorangehenden Ansprüche, wobei das Gehäuse (1) ein erstes Anschlagelement (1b) aufweist, das so angeordnet ist, dass es den Kolben (2) zurückhält, wenn er in Richtung des distalen Endes bewegt wird.

15. Vorrichtung nach Anspruch 14, wobei das Gehäuse ein zweites Anschlagelement (1c) aufweist, das so angeordnet ist, dass es das Vortriebsmittel (3) zurückhält, wenn es in Richtung des distalen Endes (1a) bewegt wird.

16. Vorrichtung nach Anspruch 1, umfassend Schutzmittel, die nach dem Injizieren manuell oder automatisch aktivierbar sind, so dass die zumindest eine Nadel (10) zumindest nach dem Herausziehen der Vorrichtung aus dem Zielgewebe geschützt ist.

## Claims

1. Device for inserting at least one hollow needle for injection or withdrawal of a solution into/from a tissue, said device comprising:
a. a casing (1), defined by a distal end (1a), which is intended to come into contact with the tissue, and an opposite proximal end,
b. a plunger (2) mounted movably inside the casing (1) and comprising:
i. a distal end (2c) intended to come close to or into contact with the tissue,
ii. said at least one hollow needle (10) intended to penetrate the tissue,
c. thrust means (3, 4) suitable for driving the plunger (2) in the direction of the distal end (1a) of the device by applying a force F1(t) thereto;
the device being **characterized**
**in that** said thrust means (3, 4) and/or said plunger (2) comprise retaining means (3a) for temporarily rigidly connecting the thrust means (3, 4) to said plunger (2) while the latter is being moved by the thrust means (3, 4), and
**in that** said retaining means (3a) are designed to free the plunger from the thrust means, allowing the thrust means to continue their travel without applying force or stress to the plunger or doing so to a limited extent when a force F2(t) of defined value greater than a non-zero threshold value, and opposite to the force F1(t), is exerted on the plunger (2).

2. Device according to Claim 1, in which the device additionally comprises pressure means intended to keep said needle at least partially inserted in the tissue and to allow at least a controlled retreat of the plunger (2) at least during the injection of the solution; said pressure means exerting a force F3(t) in the same direction as F1(t).

3. Device according to Claim 1, designed in such a way that the forces F1(t) and F2(t) are at least temporarily exerted simultaneously against the plunger (2).

4. Device according to Claim 2, designed in such a way that F3(t) is less than a maximum force F4(t) exerted against the plunger (2), F4(t) being induced by the creation of the papule following the injection of said solution (15).

5. Device according to either of Claims 2 and 4, in which said pressure means are designed in such a way as to exert the force F3(t) at least before the injection of the solution into the tissue.

6. Device according to any one of the preceding claims, in which said pressure means are designed in such a way as to activate automatically or manually.

7. Device according to any one of the preceding claims, in which the thrust means (3, 4) are designed to exert a force of a value greater than or equal to the pressure means.

8. Device according to either of Claims 2 and 4, in which F3(t) is generated exclusively or jointly by a tube (9), a spring (4a), an elastic band, a gas cartridge, compressed air, an electromagnetic force, the generation of gas by chemical reaction between at least two compounds, an elastic blade, the weight of the plunger/needle assembly (2, 10), and/or induced by the friction of the plunger against the inner walls of the casing (1).

9. Device according to any one of the preceding claims, comprising a reservoir fluidically connected to at least one needle (10).

10. Device according to any one of the preceding claims, comprising a syringe (13) with a plunger (18) intended to expel a solution (15) through the needle (10) .

11. Device according to Claim 10, in which the syringe (13) is oriented in a direction identical to or different than that defined by the movement of the needle (10).

12. Device according to any one of the preceding claims, in which the solution contained in the reservoir or in the syringe is injected into or through the tissue manually or automatically, at least after the release of the plunger from the thrust means.

13. Device according to any one of the preceding claims, comprising a tube (9) ensuring fluidic communication between a reservoir and the needle (10).

14. Device according to any one of the preceding claims, in which the casing (1) has a first stop element (1b) arranged in such a way as to retain the plunger (2) when the latter is driven towards said distal end.

15. Device according to Claim 14, in which the casing has a second stop element (1c) arranged in such a way as to retain the thruster (3) when the latter is driven towards said distal end (1a).

16. Device according to Claim 1, comprising protection means that are activatable manually or automatically after the injection, such that said at least one needle (10) is protected at least after the withdrawal of the device from the target tissue.
